(19) Europäisches Patentamt

European Patent Office

Office européen des brevets

(11) Veröffentlichungsnummer : **0 017 867**
**B1**

(12) **EUROPÄISCHE PATENTSCHRIFT**

(45) Veröffentlichungstag der Patentschrift :
05.10.83

(21) Anmeldenummer : 80101802.9

(22) Anmeldetag : 03.04.80

(51) Int. Cl.³ : **A 61 K 35/16, A 61 K 37/02,
C 07 G 7/00**

(54) **Arzneimittel zur Stimulation der Proliferation von Leberzellen und Leberschutz- und Wachstumsfaktor.**

(30) Priorität : **12.04.79 DE 2914903**

(43) Veröffentlichungstag der Anmeldung :
**29.10.80 Patentblatt 80/22**

(45) Bekanntmachung des Hinweises auf die Patenterteilung : **05.10.83 Patentblatt 83/40**

(84) Benannte Vertragsstaaten :
**CH FR GB IT LI NL SE**

(56) Entgegenhaltungen :
**CHEMICAL ABSTRACTS, Band 79, Nr. 21, 26.
November 1973, Seite 99, Nr. 122731r Columbus,
Ohio, U.S.A. L. PICKART et al. : « Tripeptide in
human serum which prolongs survival of normal
liver cells and stimulates growth in neoplastic
liver »**

**CHEMICAL ABSTRACTS, Band 71, Nr. 21, 24.
November 1969, Seite 134, Nr. 99570m Columbus,
Ohio, U.S.A. M. TIMAR et al. : « Efficacy in experimentally induced liver damage of a natural polypeptide »**

**HOPPE-SEYLER'S Z. PHYSIOL. CHEM., Zeitschrift für Physiologische Chemie, Band 356,
März 1975, Seiten 367-376 K.H. SLOTTA et al. :
« The cell growth-promoting factor »**

(73) Patentinhaber : **Max-Planck-Gesellschaft zur Förderung der Wissenschaften e.V.
Bunsenstrasse 10
D-3400 Göttingen (DE)**

(72) Erfinder : **Ruhenstroth-Bauer, Gerhard, Prof. Dr.
Spietzelbergerstrasse 11
D-8032 Gräfelfing (DE)**
Erfinder : **Goldberg, Michel
Hohenzollernplatz 8
D-8000 München 40 (DE)**

(74) Vertreter : **Dreiss, Uwe, Dr. Jur. Dipl.-Ing. M.Sc. et al
Patentanwälte Dreiss, Hosenthien & Fuhlendorf
Gerokstrasse 6
D-7000 Stuttgart 1 (DE)**

EP 0 017 867 B1

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

Arzneimittel zur Stimulation der Proliferation von Leberzellen und Leberschutz- und Wachstumsfaktor

Die Erfindung betrifft einen Leberschutz- und Wachstumsfaktor sowie ein Arzneimittel zur Stimulation der Proliferation von Leberzellen.

Es ist bereits bekannt, einen Leberwachstumsfaktor aus der *Leber* teilhepatektomierter Ratten zu extrahieren. Es handelt sich dabei um ein Protein oder Proteid, das keine für die Aktivität bedeutsame Neuraminsäure besitzt, mit einem Molekulargewicht zwischen 30 000 und 50 000 D. Die Wirkung ist organspezifisch, jedoch nicht spezies-spezifisch (Ruhenstroth-Bauer, Goldberg, Silz u. Strecker, Hoppe-Seyler's Z. Physiol. Chem. Bd. 359, S. 543-545 (April 1977) ; vgl. ferner die ältere deutsche Patentanmeldung P 28 14 981.7-41 v. 7.4.1978 derselben Anmelderin ; siehe ferner als Stand der Technik *Demetrion, A. A.* and *Levenson, S. M.,* Annual Meeting of the American Ass. for the Study of Liver Disease, Nov. 6-8, 1978, Chicago, J11., p. 959). Es ist auch Gegenstand der älteren Patentanmeldung, aus dem *Plasma* von Tieren mit teilhepatektomierter Leber einen Vorfaktor zu extrahieren, aus dem sich ein Leberwachstumsfaktor durch Behandlung mit Neuraminidase oder Neuramyltransferase abspalten lässt. Aus dieser letztgenannten Art der Gewinnung des Leberwachstumsfaktors lässt sich folgendes Wirkungsmodell ableiten : Die teilhepatektomierte Leber sendet einen irgendwie gearteten Reiz aus, der auf eine im einzelnen noch nicht bekannte Art und Weise zur Existenz des Vorfaktors im Blutplasma führt, der dann z. B. durch Neuraminidase, die im Blut teilhepatektomierter Tiere vermehrt vorhanden ist, in den eigentlichen Leberwachstumsfaktor umgesetzt wird, der zur Proliferation der Leberzellen führt.

Der Erfindung liegt die Aufgabe zugrunde, einen weiteren Leberschutz- und Wachstumsfaktor zu isolieren.

Diese Aufgabe wird dadurch gelöst, daß man aus Blutplasma durch Ansäuerung auf pH 5,0 bis 5,5 Hitzedenaturierung bei 80 bis 120 °C, Zentrifugierung und anschließende Einwirkung von Trypsin-Chymotrypsin einen Neuraminsäure- und β-Galactose-freien Blutplasma-Extrakt mit einem Molekulargewicht von etwa 1 200 D gewinnt. Die Erfindung betrifft auch ein Arzneimittel zur Stimulation der Proliferation von Leberzellen, der diesen Blutplasma-Extrakt als Wirkstoff enthält.

Die Leberwachstumsfaktoren werden danach also aus dem Blutplasma nicht teilhepatektomierter Tiere gewonnen. Die Erfindung nützt die neue Erkenntnis, daß es — neben dem oben geschilderten Regelkreis — für das Leberwachstum einen davon unabhängigen zweiten Regelkreis gibt, der durch eine stets, also nicht erst nach Teilhepatektomie, im Blutplasma vorhandene Substanz gebildet wird (Faktor aus dem Plasma von Normaltieren = NP). Durch Einwirkung von Trypsin-Chymotrypsin wird dieser Faktor in einen hochaktiven Leberwachstumsfaktor umgesetzt (Plasma von Normaltieren behandelt mit Trypsin-Chymotrypsin = NPTC). Diesen neuen Leberwachstumsfaktor kann man als Teil eines neuen « zweiten » Regelkreis bezeichnen, der von dem eingangs erörterten Regelkreis, wie er sich aufgrund der älteren Patentanmeldung P 28 14 981.7-41 ergibt, unabhängig ist (zum Nachweis siehe unten).

Man gewinnt also durch Säure-Hitze-Extraktion (NP-Extrakt) des Blutplasmas zunächst einen ersten neuen leichtaktiven Leberwachstumsfaktor. Es handelt sich dabei um eines oder mehrere Proteine. Bereits dieser hat als solcher also eine leicht proliferationsaktivierende Wirkung. Nach einer Behandlung mit Trypsin-Chymotrypsin geben diese Proteine ein oder mehrere Peptide ab, die nun außerordentlich stark proliferationsaktivierend wirken (NPTC-Extrakt).

Im Gegensatz dazu handelt es sich bei dem aus teilhepathektomierten Tieren gewonnenen Leberwachstumsfaktor gemäß der älteren Patentanmeldung P 28 14 981.7-41 um ein Protein oder Proteid, das bei einer enzymatischen Behandlung mit Trypsin-Chymotrypsin zerstört wird. Das ist ein erster Nachweis für die Unabhängigkeit dieses neuen Regelkreises von dem oben erwähnten ersten Regelkreis.

Daß der NP-Extrakt, der aus Blutplasma durch Ansäuerung und Hitzedenaturierung gewonnen wird, bereits eine gewisse Proliferations-Aktivität aufweist, kann auf eine Eigenschaft der Proteine selbst zurückzuführen sein oder aber darauf, daß schon im NP-Extrakt, möglicherweise an die Proteine gebunden, die im NPTC-Faktor enthaltenen Peptide in gewisser Menge vorhanden sind.

Der noch nicht mit Trypsin-Chymotrypsin behandelte NP-Extrakt normaler Tiere weist bereits eine gewisse Proliferationsaktivität auf, die über derjenigen der (NaCl-)Kontrollwerte liegt. Die Aktivität des mit Trypsin-Chymotrypsin behandelten NPTC-Extraktes liegt erheblich höher. Peptid : Peptidyl-Hydrolasen wie Trypsin-Chymotrypsin spalten typischerweise Peptidverbindungen bzw. -ketten in einzelne Peptide auf. Eine Behandlung dieses hochwirksamen Leberwachstumsfaktors NPTC mit Pronase, einem Enzym, das Peptide weiter abbaut, ergibt einen vollkommenen Verlust der Proliferationsaktivität. Daraus ergibt sich, daß der neue Leberwachstumsfaktor ein Peptid ist.

Daß es sich bei diesem neuen Regelkreis um einen solchen handelt, der von dem bekannten Regelkreis unabhängig ist, ergibt sich neben dem oben erwähnten Nachweis ferner daraus, daß eine Behandlung des neuen Leberwachstumsfaktors mit Neuraminidase oder Beta-Galaktosidase seine Aktivität nicht beeinflußt. Es wird also nicht, wie bei dem bereits bekannten « ersten » Leberwachstumsfaktor, sofern er aus Plasma gewonnen wird, durch Behandlung mit Neuraminidase eine Neuraminsäure abgespalten und damit der Rest aktiviert.

Da ferner Neuraminsäure meist an Beta-Galaktose gekoppelt ist, die nach Abspaltung der Neuraminsäure von entsprechenden Rezeptoren in der Leber aufgenommen wird, müsste ein Faktor auf der Grundlage dieses bekannten Modells inaktiv werden, wenn die Beta-Galaktose durch Beta-Galaktosidase abgespalten wird. Das ist jedoch bei dem neuen Leberwachstumsfaktor nicht der Fall.

Wie im einzelnen weiter unten noch darzustellen sein wird, läßt sich der im NP-Extrakt enthaltene Wirkstoff qualitativ zwar als Protein oder Proteid kennzeichnen ; seine wichtigste Charakterisierung ist aber darin zu sehen, daß bei Einwirkung von Trypsin-Chymotrypsin die wesentlich genauer charakterisierbaren Wirkstoffe, die im NPTC-Extrakt enthalten sind, entstehen.

Der neue Leberwachstumsfaktor wird wie folgt gewonnen :

Man entnimmt das Blutplasma normaler Ratten. Die Versuche wurden mit weiblichen 95-105 g schweren SPF-Wistar-Ratten (Institut für Strahlen-Umweltforschung, Neuherberg/München) durchgeführt. Nach der Tötung der Tiere wurden diese entblutet und das Blutplasma wie folgt gewonnen und aufbereitet : Das heparinisierte Vollblut wird 20 min. bei 4 000 g zentrifugiert und das Plasma abpipettiert.

Das gewonnene Blutplasma wurde mit einer Salzsäure (HCl-)Lösung in der Konzentration von 0,1 N auf den pH-Wert 5,5 gebracht. Diese Ansäuerung ist ein wichtiges Selektionsmittel zur Abspaltung einer großen Anzahl von Proteinen : Sie werden ausgefällt und damit dem weiteren Konzentrations- bzw. Isolationsprozeß entzogen. Nach Abscheidung der ausgefällten und damit unwirksam gewordenen Substanzen wurde die Restsubstanz bei einer Temperatur von 95 °C für die Dauer von 20 Minuten hitzedenaturiert. Damit werden weitere Bestandteile des Plasmas, und zwar solche, die bei dieser Hitze und bei diesem pH-Wert nicht beständig sind, ausgefällt.

Durch die Ansäuerung auf 5,5 pH und die Hitzedenaturierung wird ein großer Anteil der Bestandteile des Plasmas entfernt. Anschließend erfolgt eine Zentrifugierung für die Dauer von 15 Minuten mit 4 000 g (Minifuge Christ Osterode/Hartz). Der Überstand enthält somit nur diejenigen der insgesamt ursprünglich im Blutplasma enthaltenen Wirkstoffe, die bei pH = 5,5 und 95 °C stabil sind, diese jedoch in konzentrierter Form. Um auch noch diejenigen Teile der Wirkstoffe zu erfassen, die evtl. in den bei der Zentrifugierung enthaltenen Niederschlägen enthalten sind, wurden die Niederschläge wiederum mit aqua bidest. auf das ursprüngliche Volumen aufgefüllt, erneut einer Ansäuerung von pH = 5,5 und einer Hitzedenaturierung bei 95 °C unterzogen und zentrifugiert. Dieser Vorgang wurde insgesamt zweimal wiederholt. Damit hat man den NP-Extrakt erhalten.

Die Überstände (NP-Extrakt) dieser insgesamt drei Zentrifugationsvorgänge wurden zusammengegeben und der folgenden Trypsin-Chymotrypsin-Behandlung unterzogen.

Von den Überständen werden 150 mg in 20 ml aqua bidest. gelöst, auf pH 7,6 gebracht und bei 30 °C für zwei Stunden mit jeweils 80 U Trypsin, reinst. und 90 U $\alpha$-Chymotrypsin, reinst. (Serva, Heidelberg) inkubiert.

Zur Inaktivierung der restlichen Enzymaktivitäten wurde dann 30 Minuten bei 95 °C inkubiert und zentrifugiert. Man erhielt derart den den Leberwachstumsfaktor enthaltenden NPTC-Extrakt.

Danach wurde die Lösung lyophilisiert, in 2 ml einer 0,9 % NaCl-Lösung aufgenommen und normalen Ratten intraperitonal (i. p.) injiziert. Kontrolltieren wurde dieselbe Menge einer 0,9 %igen NaCl-Lösung i. p. injiziert.

Die Messung der Proliferation der Leberzellen nach Injektion des NPTC erfolgt durch Messung der DNA-Synthese, indem in die DNA eingebaute radioaktive Substanzen gemessen wurden, nämlich $^3$H-Methylthymidin (spezielle Aktivität von 25 Ci/mmol ; Radiochem. Center, Amersham).

Den Versuchstieren und den Kontrolltieren wurde 19 Stunden nach der Injektion des NPTC 50 $\mu$ Ci $^3$H-Methylthymidin injiziert. Nach einer Stunde wurden die Tiere getötet. Die Leber wurde entfernt und bei $-$ 20 °C aufbewahrt.

Danach erfolgte eine Extraktion der DNA der Leber nach Weinbren, K. u. Woodward, E. (Br. J. exb. Path. 45, 442-449 (1964)). Ein Teil dieses Extraktes wurde zu einer Radioaktivitätsmessung verwendet. Dazu wurden 1,5 ml PCA-(Perchloressigsäure) haltigen Lösung mit 0,5 ml NaOH in der Konzentration 1N neutralisiert. Die entstehende Lösung wurde in einem Scintilationsgläschen mit 5 ml Triton X und 10 ml Toluol (0,6 PPO ; PPO = 1,5-Diphenylloxazol) versetzt.

Mit Hilfe eines Liquid-Scintilationszählers (Intertechnique, Paris) wurde dann die Radioaktivität als Anzahl der Zerfälle pro Minute (Zpm) gemessen. Ein weiterer Teil des DNA-Extraktes wurde zur Messung der DNA-Konzentration nach Burton (Biochem. J. 62, S. 315-323 (1956)) verwendet. Man erhält auf diese Weise als Maß für die DNA-Synthese die spezifische Aktivität in Zpm/$\mu$g DNA.

Die mit dem NPTC-Extrakt injizierten normalen Ratten zeigten unter den genannten Versuchsbedingungen im Durchschnitt eine mittlere spezifische Aktivität von 372 $\pm$ 95 Zpm/$\mu$g DNA (Anzahl der Versuchstiere : n = 8).

Die mit dem NP-Extrakt injizierten normalen Ratten zeigten unter denselben Bedingungen im Durchschnitt eine mittlere spezifische Aktivität von 170 $\pm$ 36 Zpm/$\mu$g DNA (n = 6).

Bei den mit physiologischer Kochsalzlösung injizierten normalen Ratten ergab sich ein Wert von 105 $\pm$ 22 Zpm/$\mu$g DNA (n = 8).

Der aus dem normalen Blutplasma gewonnene NP-Extrakt ohne Trypsin-Chymotrypsin-Behandlung zeigt bereits eine erhöhte Aktivität. Der NPTC-Extrakt zeigt eine Steigerung um den Faktor 3,5 mit hoher statistischer Sicherheit (p $\ll$ 0,01).

Dieser Nachweis bedeutet, daß die i. p.-Injektion des NPTC-Extraktes bei normalen Tieren zu einer erheblichen Steigerung der DNA-Synthese führt, die ihrerseits eine notwendige Voraussetzung einer Zellteilung und damit eines Leberwachstums durch Zellteilung ist.

Ein weiterer Versuch ergab, daß der NPTC-Extrakt auch in Kulturen von Hepatozyten adulter Ratten proliferationsaktiv ist. Diese Kulturen wurden wie folgt gewonnen :

Aus Ratten mit einem Gewicht von 350-450 g wurden Hepatozyten mit Collagenase nach der Methode von G. Williams (in vitro, 13, 809 (1977)) isoliert. Die Zellen wurden mit jeweils 10 ml L-15 Medium (Boehringer Mannheim) zweimal gewaschen. Danach wurden sie in L-15-Medium aufgeschwemmt, die Endkonzentration betrug 2-4 × $10^5$ Zellen/ml. Jeweils 1 ml dieser Suspension kam in eine Petrischale, dazu wurden je 100 μl fötales Kälberserum zugefügt. Nach einer Inkubationszeit von 4 Stunden (37,5 °C, 100 % $O_2$) wurden je Schale 5-7 μg eines NPTC-Extraktes bzw. bei den Kontrollen physiologische NaCl-Lösung hinzugefügt. Nach weiteren 19 Stunden Inkubation wurde für eine Stunde mit jeweils 0,5 μCi $^3$H-Thymidin markiert. Nach 6-maligem Waschen mit NaCl-Lösung wurde der Einbau mit eiskalter 7 % TCA-Lösung gestoppt, die Zellen auf Glasfaserfilter abgesaugt und mit TCA und anschließend mit Äthanol nachgewaschen. Die Filter wurden bei 120 °C getrocknet und nach oben geschilderter Methode im Liquid-Scintilationszähler gemessen.

Im Vergleich zu Kontrollen zeigte sich bei den Kulturen, zu denen NPTC zugegeben war, ein um den Faktor 3-4 vermehrter Einbau von Thymidin im Vergleich mit den Kontrollkulturen.

Qualitativ läßt sich der gefundene Leberwachstumsfaktor wie folgt charakterisieren :

Eine Behandlung des NPTC-Extraktes mit Pronase führte zur Inaktivität : Dazu wurde ein NPTC-Extrakt in 10 ml aqua bidest. gelöst, auf pH 7,5 gebracht und bei 37 °C für zwei Stunden mit 100 U Pronase P (Sigma, München) inkubiert. Zur Inaktivierung der restlichen Enzymaktivität wurde dann 30 min. bei 95 °C inkubiert und zentrifugiert. Danach wurde die Lösung lyophilisiert, in 2 ml 0,9 % NaCl-Lösung aufgenommen und normalen Ratten injiziert.

Es ergab sich eine Proliferationsrate von 75 ± 9 Zpm/μg DNA. Da einerseits die hohe Aktivität durch eine Trypsin-Chymotrypsin-Behandlung erst hergestellt wird, andererseits durch eine Behandlung mit Pronase zerstört wird, läßt sich daraus der Schluß ziehen, daß es sich um ein Peptid handelt.

Der NPTC-Extrakt wurde ferner mit Neuraminidase und mit Galaktosidase behandelt :

Zur Behandlung mit β-Galaktosidase wurde ein NPTC-Extrakt in 10 ml aqua bidest. gelöst, auf pH 7,3 gebracht und bei 37 °C für eine Stunde mit 5 U β-Galaktosidase (Sigma, München) inkubiert. Zur Inaktivierung der restlichen Enzymaktivität wurde dann 30 min. bei 95 °C inkubiert und zentrifugiert. Danach wurde die klare Lösung lyophylisiert, in 2 ml 0,9 % NaCl-Lösung aufgenommen und normalen Ratten injiziert.

Zur Behandlung mit Neuraminidase wurde ein NPTC-Extrakt in 10 ml aqua bidest. gelöst, auf 5,5 gebracht und bei 37 °C für eine Stunde mit 250 U Neuraminidase (Behringwerke AG, Marburg) inkubiert. Zur Inaktivierung der restlichen Enzymaktivität wurde dann 30 min. bei 95 °C inkubiert und zentrifugiert. Danach wurde die Lösung lyophilisiert, in 2 ml 0,9 % NaCl-Lösung aufgenommen und normalen Ratten injiziert.

Sowohl nach der Enzymbehandlung mit β-Galaktosidase als auch nach Neuraminidase-Behandlung änderte sich nicht die hohe Proliferationsaktivität des behandelten NPTC-Extraktes.

Zur Bestimmung des Molekulargewichts wurde eine G 15-Chromatographie durchgeführt. Als Säulenmaterial wurde Sephadex® G 15 verwendet (Säulenvolumen 121 ml). Nach dem Äquilibrieren der Säule mit 50 mM TRIS-Puffer, pH 7,6, wurde ein NPTC-Extrakt, gelöst in 2 ml desselben Puffers, aufgetragen. Die Flußrate betrug 11 ml/h. Es wurden 5 ml-Fraktionen gesammelt und die $OD_{280}$ gemessen. Die einzelnen Proteingipfel wurden gesammelt, lyophilisiert, in 2 ml aqua bidest. aufgenommen und normalen Ratten injiziert. Zur Bestimmung des Molekulargewichts wurden folgende Referenzsubstanzen unter denselben Bedingungen auf die Säule aufgetragen : Glutathion (MG 307), NAD (MG 663), 7er Peptid (MG 981). Für den aktiven Proteingipfel ergab sich ein Molekulargewicht von ca. 1 200.

Mit den beschriebenen Methoden wurde auch ein NPTC-Extrakt aus menschlichem Blutplasma hergestellt. Der derart aus Humanplasma gewonnene NPTC-Extrakt wurde Ratten i.p. injiziert. Nach der Aufarbeitung ergab sich bei den Ratten eine Proliferationsrate von 410 ± 112 Zpm/μg DNA (n = 5).

Daraus ist der Schluß zu ziehen, daß der NPTC-Faktor nicht spezies-spezifisch ist. Das läßt dann wiederum mit hoher Wahrscheinlichkeit aufgrund der Versuche mit Ratten den Schluß zu, daß der Faktor auch beim Menschen dieselbe Wirkung hat.

Eine Untersuchung von Milz und Niere der Ratten nach einer Injektion des NPTC-Extraktes ergab Proliferationsraten von 307 ± 66 Zpm/μg DNA für Milz (Kontrolle 354 ± 31) und 51 ± 13 Zpm/μg DNA für Niere (Kontrolle 43 ± 10) (n = 4).

Es ergab sich also keine feststellbare Veränderung. Daraus ist der Schluß zu ziehen, daß der gefundene Leberwachstumsfaktor organspezifisch ist.

Für die Herstellung wurde oben u. a. die Ansäuerung auf pH = 5,5 und die Hitzedenaturierung bei ca. 95 °C angegeben. Das ist im Sinne einer möglichst effektiven Gewinnung zu verstehen. Es hat sich gezeigt, daß der Faktor auch noch bei 120 °C stabil ist, wenngleich die Gewinnung am besten in der dargestellten Weise bei 95 °C erfolgt. Auch eine Hitzedenaturierung bei nur 80 °C kann ein bereits in gewissem Umfang brauchbares Ergebnis liefern.

Dasselbe gilt für den pH-Wert. Abweichungen (also etwa pH = 5,0 oder 5,3) führen dazu, daß man weniger Substanzen ausfällt ; man erhält also einen weniger reinen Extrakt, der jedoch auch schon eine gewisse Wirksamkeit hat. Im Bereich von pH = 5,0 bis 5,5 haben sehr viele Proteine ihren isoelektrischen Punkt, so daß gerade dieser Bereich für das Ausfällen vieler Proteine günstig ist.

**Ansprüche**

1. Leberschutz- und Wachstumsfaktor, dadurch gekennzeichnet, daß er durch einen Neuraminsäure-freien sowie Beta-Galactose-freien Blutplasmaextrakt mit einem Molukulargewicht von etwa 1 200 D gebildet wird, der aus Blutplasma durch Ansäuerung auf pH 5,0 bis 5,5, Hitzedenaturierung bei 80 bis 120 °C, Zentrifugierung und anschließende Einwirkung von Trypsin-Chymotrypsin gewonnen wird.

2. Arzneimittel zur Stimulation der Proliferation von Leberzellen, dadurch gekennzeichnet, daß es als Wirkstoff einen Neuraminsäure-freien sowie Beta-Galactose-freien Blutplasmaextrakt mit einem Molekulargewicht von etwa 1 200 D enthält, der aus Blutplasma durch Ansäuerung auf pH 5,0 bis 5,5, Hitzedenaturierung bei 80 bis 120 °C, Zentrifugierung und anschließende Einwirkung von Trypsin-Chymotrypsin gewonnen wird.

**Claims**

1. Liver protection and growth factor, characterized by being provided by a neuraminic-acid-free and beta-galactose-free blood plasma extract with a molecular weight of 1 200 D, obtained from blood plasma by acidification to a pH-level of 5,0 to 5,5, heat denaturation at 80° to 120 °C, centrifugation and subsequent treatment with trypsin-chymotrypsin.

2. Pharmaceutical drug for stimulating the proliferation of liver cells, characterized in that as the effective ingredient it contains a neuramidic-acid-free and beta-galactose-free blood plasma extract, obtained from blood plasma by acidification to a pH-level of 5,0 to 5,5 heat denaturation at 80° to 120 °C, centrifugation and subsequent treatment by trypsin-chymotrypsin.

**Revendications**

1. Facteur de protection et de développement du foie caractérisé en ce qu'il est formé d'un extrait du plasma du sang exempt d'acide de neuramine ainsi que de β-galactose ayant un poids moléculaire d'environ 1 200 D, lequel est obtenu du plasma sanguin par acidification à pH de 5,0 à 5,5, dénaturation à la chaleur à 80 à 120 °C, centrifugation et finalement action de trypsine-chymotrypsine.

2. Médicament pour la stimulation de la prolifération des cellules du foie caractérisé en ce qu'il contient, comme agent actif, un extrait de plasma sanguin exempt d'acide de neuramine ainsi que de β-galactose, ayant un poids moléculaire d'environ 1 200 D, qui est obtenu du plasma sanguin, par acidification à pH 5,0 à 5,5, dénaturation à la chaleur à 80 à 120 °C, centrifugation et finalement action de la trypsine-chymotrypsine.